# EUROPÄISCHE PATENTSCHRIFT

(11) **EP 1 170 357 B1**
(45) Veröffentlichungstag und Bekanntmachung des Hinweises auf die Patenterteilung: **02.03.2005**
(21) Anmeldenummer: 00125062.0
(22) Anmeldetag: 17.11.2000
(51) Int. Cl.: C12M 1/113

(54) **Feststoff-Zudosier-Vorrichtung an einem Fermenter einer Biogasanlage**
Apparatus for supplying solid material to a digester of a biogas production plant
Dispositif d'alimentation en matières solides pour un fermenteur d'une installation de production de biogaz

(30) Priorität: 06.07.2000 DE 20011785 U
(43) Veröffentlichungstag der Anmeldung: 09.01.2002
(73) Patentinhaber: U.T.S. Umwelt-Technik-Süd GmbH, 84419 Obertaufkirchen (DE)
(72) Erfinder: Bürger, Adam, 84405 Grüntegernbach (DE)
(74) Vertreter: Neubauer, Hans-Jürgen, Dipl.-Phys.

(56) Entgegenhaltungen:
- DE-C- 4 211 221
- FR-A- 2 612 834
- FR-A- 2 709 760
- GB-A- 2 282 337
- US-A- 2 159 258

## Beschreibung

Die Erfindung betrifft eine Feststoff-Zudosier-Vorrichtung an einem Fermenter einer Biogasanlage nach dem Oberbegriff des Anspruchs 1.

Ein Fermenter zur anaeroben Fermentation von organischen Stoffen einer Biogasanlage ist aus der DE 196 21 914 C1 bekannt. Bei bekannten Anlagen wird vorrangig Flüssigmist/Gülle fermentiert, die dem Fermenter über Pumpsysteme und Rohrleitungen zugeführt werden.

Es ist auch allgemein bekannt, organische Feststoffe, wie Holzabfälle, Feldfruchtabfälle, etc. beim Fermentationsvorgang zuzudosieren. Eine solche Zudosierung erfolgt in an sich bekannter Weise durch einen verschließbaren Einwurfschacht, der in der Art eines Tauchrohrs bis unter den Flüssigkeitsspiegel des Fermenters geführt ist, wo regelmäßig eine Schrägfläche als Rutsche zum eigentlichen Fermentervolumen angeordnet ist. Ersichtlich entstehen hier Probleme bei der Zuführung von größeren Zudosiermengen, insbesondere wenn diese aufschwimmen oder plötzlich in den Fermenterbehälter absacken. Zudem ist bei jeder Zudosierung der Schacht zu öffnen, wodurch auch im oberen Schachtbereich gebildetes Biogas mit erheblicher Geruchsbelästigung freigesetzt wird. Insgesamt ist eine solche Zudosierung nur für gelegentlich anfallende kleinere Zudosiermengen geeignet.

In der Zukunft ist es geplant, im landwirtschaftlichen Bereich einen Teil der volkswirtschaftlich erforderlichen Energie durch Fermentation großer Mengen nachwachsender biologischer Rohstoffe zu schaffen.

Aus der US 2,159,258 ist ein Aufschlussverfahren bzw. eine Aufschlussvorrichtung bekannt, bei der das aufzuschließende Faser- oder Zeilmaterial möglichst vollständig von Sauerstoff oder Luft befreit werden soll, bevor es in die eigentliche Aufschlussvorrichtung gelangt. Dazu wird das Faser- oder Zeilmaterial gewässer und anschließend mittels einem Kolben durch ein perforiertes und sich in Schieberichtung des Kolbens verjüngendes Zylinderrohrstück gepresst, das zur Ausbildung einer Doppelwandanordnung von einer einen Spaltabstand dazu aufweisenden äußeren Zytinderrohrwand ummantelt ist. In dieses sich verjüngende, innere Zylinderrohrstück werden der Sauerstoff und die Luft zusammen mit einem großen Anteil von Wasser von dem Faser- oder Zellmaterial entfernt, das über die Perforationslöcher des inneren Zylinderrohrstückes in den doppelwandigen Außenbereich gelangt und dort abgezogen wird. Das so entgaste und auch entwässerte Material gelangt dann in die eigentliche Aufschlussvorrichtung, die durch ein ebenfalls eine Vielzahl von Perforationslöchern aufweisendes drehbar gelagertes Perforationsrohr gebildet ist, das wiederum zur Ausbildung einer Doppelwandanordnung von einer ortsfesten und einen Spaltabstand zu dem drehbar gelagerten inneren Perforationsrohr aufweisenden äußeren Zylinderrohr umgeben ist. In dieses äußere Zylinderrohr mündet eine Zuführleitung, mittels der zur Durchführung der eigentlichen Aufschlussreaktion Flüssigkeit, wie z. B. Sulfite, unter Druck zugeführt werden kann. Diese zugeführte Flüssigkeit soll das in dem perforierten Innenrohr aufgenommene und von Gasen befreite Faser- oder Zellmaterial im Laufe des Transportes durch die eigentliche Aufschlussvorrichtung auslaugen. Die dem äußeren Zylinderrohr zugeführte Flüssigkeit gelangt dabei über die Perforationslöcher des Innenrohres in Kontakt mit dem darin angeordneten Feststoffmaterial und kann auch wieder über die Perforationslöcher in den Spaltbereich der Doppelwandanordnung austreten. Die Perforationslöcher sind so gewählt, dass keinerlei Feststoff aus dem Innenrohr heraus in den das Innenrohr umgebenden Flüssigkeitsraum gelangen kann. Eine Zudosierung von Feststoffen findet somit hier nicht statt.

Gleiches trifft auf die GB 2 282 337 zu, die einen zur US 2,159,258 ähnlichen Aufbau zeigt, bei dem ebenfalls keine unmittelbare Zudosierung von Feststoffen in die in einen Fermenter aufgenommene Flüssigkeit erfolgt, sondern lediglich ein Transport von Feststoffen über ein Perforationsrohr durch eine in einem Behälter aufgenommene Flüssigkeit hindurch. Die Zudosierung von Feststoffen in die Flüssigkeit ist somit auch hier nicht vorgesehen.

Aufgabe der Erfindung ist es, eine Feststoff-Zudosier-Vorrichtung für einen Fermenter einer Biogasanlage zu schaffen, mit der eine effektive Zudosierung von Feststoffen, insbesondere von großen Zudosiermengen, in eine in einem Fermenterbehälterinnenraum aufgenommene Flüssigkeit so vorgenommen werden kann, dass ein plötzliches Absacken bzw. ein unkontrolliertes Aufschwimmen von zudosierten Feststoffen in der Flüssigkeit verhindert werden kann.

Diese Aufgabe wird mit den Merkmalen des Anspruchs 1 gelöst.

Gemäß Anspruch 1 besteht die Feststoff-Zudosiervorrichtung aus einem Einwurfschacht mit anschließendem Zwischensammelraum für organische Feststoffe und einem nachgeordneten Förderrohr sowie einer Linearfördereinrichtung. Das Förderrohr ist zudem dicht mit einer Fermenterzuführöffnung verbunden, so dass die geförderten organischen Feststoffe in den Fermenterbehälter zuführbar sind, ohne dass Biogas aus dem Fermenterbehälter durch den Förderrohranschluss und um diesen herum austreten kann. Die Linearfördereinrichtung ist aus einer Kolbenpresse gebildet, deren Pressenstempel im Bereich des Zwischensammelraums angetrieben bewegt wird dergestalt, dass für eine chargenweise Förderung im Zwischensammelraum enthaltene organische Feststoffe mittels des Pressenstempels in und durch das Förderrohr gepresst und in die Flüssigkeit im Fermenterbehälter eingebracht werden. Bei zurückgezogenem Pressenstempel kann somit der Zwischensammelraum und gegebenenfalls der damit verbundene Einwurfschacht mit organischen Feststoffen gefüllt werden. Anschließend kann der Pressenstempel durch den Zwischensammelraum geschoben werden, wodurch dieser ausgeräumt wird und die darin vorher enthaltenen organischen Feststoffe in das Förderrohr gepresst werden. Der Zwischensammelraum wirkt dabei in der Art eines Zylinderraums für den Pressenstempel.

Mit einer solchen Feststoff-Zudosiervorrichtung können einfach, bequem und mit genauer Dosierung auch große Zudosiermengen in den Fermenterbehälter eingebracht werden. Beispielsweise können organische Feststoffe mit einem Gabellader in den Einwurfschacht eingeworfen und durch Betätigung der Kolbenpresse durch das Förderrohr in den Fermenterbehälter befördert werden. Die bisherigen Probleme mit Tauchrohr-Zuführeinrichtungen, durch ein Aufschwimmen von Zudosierstoffen oder ein mögliches plötzliches Absacken und Eindringen großer Zudosiermengen mit ungünstigen Auswirkungen auf den laufenden Fermentationsprozess und Druckerhöhungen im Fermenterbehälter, können hier nicht mehr auftreten.

Es ist darauf zu achten, dass über das Förderrohr weder Biogas entweichen noch Flüssigkeit aus dem Fermenter herauslaufen kann. Je nach verarbeiteten organischen Feststoffen kann gegebenenfalls eine dichte Absperrung im Förderrohr bereits durch den sich darin befindenden und jeweils weiter geschobenen Pfropfen aus organischen Feststoffen hergestellt sein. Je nach den speziellen Gegebenheiten kann dabei nach Anspruch 2 das Förderrohr mit seiner Auslassöffnung im oberen Fermenterinnenbereich oberhalb und/oder unterhalb des Flüssigkeitsspiegels enden. Besonders vorteilhaft ist das Förderrohr im Endbereich nach unten abgebogen und erstreckt sich mit seiner Auslassöffnung in der Art eines Schwanenhalses bis unterhalb des üblichen Flüssigkeitsspiegels. Damit wird sicher ein Austritt von Biogas und ein Zurückströmen von Flüssigkeit aus dem Förderrohr verhindert.

Mit den Merkmalen der Ansprüche 3 und 4 werden alternative Führungen des Förderrohrs angegeben, mit denen jeweils sichergestellt ist, dass die Feststoff-Zudosier-Vorrichtung nicht einfrieren kann.

Je nach der Art der zudosierten organischen Feststoffe, dem Durchmesser des Förderrohrs, der Länge des Förderrohrs und der darin enthaltenen Umlenkungen, des Pressendrucks und weiterer Parameter ergibt sich im konkreten Fall ein mehr oder weniger gut förderbarer Pfropfen aus gepressten organischen Feststoffen im Förderrohr. Um die Förderfähigkeit eines solchen Pfropfens auch bei ungünstigen Verhältnissen zu gewährleisten und zu verbessern, wird mit Anspruch 5 vorgeschlagen, dass der Durchmesser des Förderrohrs ausgehend von der Kolbenpresse zum Fermenterbehälter hin ansteigt und größer wird. Ein solcher Durchmesseranstieg kann kontinuierlich oder aus Gründen einer einfacheren Herstellung stufenförmig erfolgen. Das Förderrohr kann dabei zylindrisch oder auch im Querschnitt eckig ausgebildet sein. Durch die allmähliche Durchmesservergrößerung im Verlauf des Förderrohrs steht dem Pfropfen im Förderrohr ein entsprechend größeres Volumen zur Verfügung, so dass die Reibung verringert und die Förderbarkeit verbessert wird.

Mit den Merkmalen des Anspruchs 6 wird vorgeschlagen, dass im Verlauf des Förderrohrs wenigstens eine Wasser-Spüldüse für die Anfeuchtung eines im Förderrohr enthaltenen Pfropfens aus organischen Feststoffen angebracht ist. Auch durch eine solche Spülung und Anfeuchtung kann die Förderbarkeit verbessert werden. Gegebenenfalls können solche Wasser-Spüldüsen auch zu Reinigungszwecken verwendet werden.

Vorteilhaft wird gemäß Anspruch 7 die Kolbenpresse als Hydraulikpresse mit einem bewährten Hydraulikantrieb ausgeführt, wodurch zudem Probleme mit dem Explosionsschutz vermieden werden. Auch alle anderen Antriebe und Stellglieder der gesamten Anlage können vorteilhaft entsprechend hydraulisch angetrieben werden.

Zweckmäßig ist nach Anspruch 8 der Pressenstempel wenigstens so lang wie die entsprechende Länge des Zwischensammelraums, so dass beim Zurückziehen des Pressenstempels keine Feststoffe hinter den Pressenstempel gelangen können und dadurch auch keine Gefahr einer Verklemmung besteht. Bei einer solchen Kolbenpresse kann vorteilhaft auf funktionsfähige und auf dem Markt erhältliche Pressen zurückgegriffen werden, wobei eine hohe Pressenkraft mit relativ wenig Energie erreichbar ist.

In einer besonders bevorzugten Weiterbildung nach Anspruch 9 ist im Einwurfschacht oberhalb der Linearfördereinrichtung und gegebenenfalls mit dieser zusammenwirkend wenigstens eine angetriebene Zerkleinerungs- und/oder Mischeinrichtung angeordnet. Solche Zerkleinerungs- und/oder Mischeinrichtungen können z. B. an sich bekannte Häcksler sein. Damit ist es möglich, eine Vielzahl von organischen Feststoffen, wie Äste, Zweige, Stroh, Rübenblätter, etc. ohne aufwendige vorherige Trennung oder Aufbereitung in den Einwurfschacht einzufüllen, da dort eine geeignete Aufbereitung durch Zerkleinern und Mischen unmittelbar vor der Förderung in den Fermenter erfolgt.

Ein Problem bei der Zudosierung von organischen Feststoffen besteht darin, dass beim Betrieb der Biogasanlage nach jeweils relativ kurzen Zeiten nur relativ kleine Zudosiermengen chargenweise dem Fermenterbehälter zugeführt werden können. Der vorstehend angegebene Einwurfschacht mit angeschlossenem Zwischensammelraum kann für eine Bevorratung großer Mengen an organischen Feststoffen nicht beliebig vergrößert werden, da bei einem großen Einwurfschacht die Gefahr von Störungen durch ein blockiertes Nachrutschen und Probleme beim Ausschieben der Zudosiermenge aus dem Zwischensammelraum durch den Pressenstempel bestehen. Ohne zusätzliche Maßnahmen muss daher der Einwurfschacht ungünstig oft von einer Bedienperson nachgefüllt werden.

Daher wird mit Anspruch 10 vorgeschlagen, dass ein zusätzlicher, relativ großer Vorratsbehälter für größere Mengen an organischen Feststoffen vorgesehen ist, mit einer Zuführeinrichtung zum Einwurfschacht und zum Zwischensammelraum bzw. zur Kolbenpresse. Der Vorratsbehälter kann dabei stationär angebracht sein und jeweils vor Ort aufgefüllt werden oder auch bereits zum Antransport von organischen Feststoffen zur Biogasanlage verwendet werden.

Dazu kann ein solcher Vorratsbehälter selbstfahrbar oder in der Art eines Containers ausgebildet sein.

Die Zufuhreinrichtung eines solchen Vorratsbehälters kann nach Anspruch 11 in einer einfachen und funktionsfähigen Anordnung aus einer Bodenfördereinrichtung am Boden des Vorratsbehälters, einem sog. Kratzboden, bestehen. Dieser kann beispielsweise durch ein Förderband mit darauf angebrachten Mitnehmern oder durch nebeneinanderliegende Walzen, die in Richtung eines Behälterspalts drehen, gebildet sein. Aus einem solchen Behälterspalt können dann organische Feststoffe unmittelbar oder mittelbar über eine weitere Fördereinrichtung dem Einwurfschacht zugeführt werden.

In einer alternativen Ausführungsform nach Anspruch 12 enthält ein solcher großer Vorratsbehälter eine Zerkleinerungs- und/oder Mischeinrichtung, die aus einer Förderschnecke und aus wenigstens weiteren zwei, jeweils seitlich der Förderschnecke achsparallel angeordneten Zusatzschnecken mit einer Drehrichtung entgegen der Förderschnecken-Drehrichtung besteht. Damit wird vorteilhaft eine Zerkleinerung und Mischung im Vorratsbehälter durchführbar dergestalt, dass zwischen den Einzelschnecken größere organische Feststoffteile zerrieben und zerrissen werden. Mit der Förderschnecke können dann durch eine zugeordnete Behälteröffnung organische Feststoffe unmittelbar oder mittelbar über eine weitere Fördereinrichtung dem Einwurfschacht zugeführt werden.

Falls erforderlich, können nach Anspruch 13 im Förderrohr betätigbare Absperreinrichtungen angebracht werden, die beispielsweise ein Rückströmen von Biogas durch einen Feststoffpfropfen geringer Konsistenz verhindern können.

Die Biogastechnik wird von einem Betreiber nur angenommen werden, wenn die damit verbundenen Arbeiten in relativ weit auseinanderliegenden Zeitabständen mit geringem Aufwand durchführbar sind. Dies kann in Verbindung mit der erfindungsgemäßen Feststoff-Zudosier-Vorrichtung, insbesondere bei Verwendung eines großen Vorratsbehälters und einer programmierbaren Steuereinheit nach Anspruch 14 realisiert werden. Mit einer solchen Steuereinheit kann durch Erfassung von Betriebsparametern, insbesondere des Gasdrucks im Fermenterbehälter und der Temperatur, sowie ggf. weiterer Parameter ein geeigneter Zeitpunkt für eine Zudosierung ermittelt und diese automatisiert durch Ansteuerung des Antriebs für den Pressenstempel und ggf. von Absperreinrichtungen und Sprühdüsen durchgeführt werden. Das Erfordernis einer Überprüfung und der Nachfüllung eines Vorratsbehälters kann damit auf mehrtägige Zeitabstände ausgedehnt werden, was die Akzeptanz der Biogastechnik, insbesondere im landwirtschaftlichen Bereich, vorteilhaft erhöht.

Anhand einer Zeichnung wird die Erfindung näher erläutert.

Es zeigen:
- Fig. 1: eine schematische Schnittansicht durch eine Feststoff-Zudosier-Vorrichtung an einem Fermenter einer Biogasanlage,
- Fig. 2: eine schematische Schnittdarstellung einer alternativen Ausführungsform einer Feststoff-Zudosier-Vorrichtung an einem Fermenter eine Biogasanlage,
- Fig. 3: eine schematische Schnittdarstellung durch eine weitere alternative Ausführungsform einer Feststoff-Zudosier-Vorrichtung mit einem Vorratsbehälter und Zuführeinrichtung,
- Fig. 4: eine schematische Schnittdarstellung entlang der Linie A-A der Fig. 3.
- Fig. 5: eine weitere alternative Ausführungsform zu Fig. 3, und
- Fig. 6: eine Schnittdarstellung entlang der Linie B-B der Fig. 5.

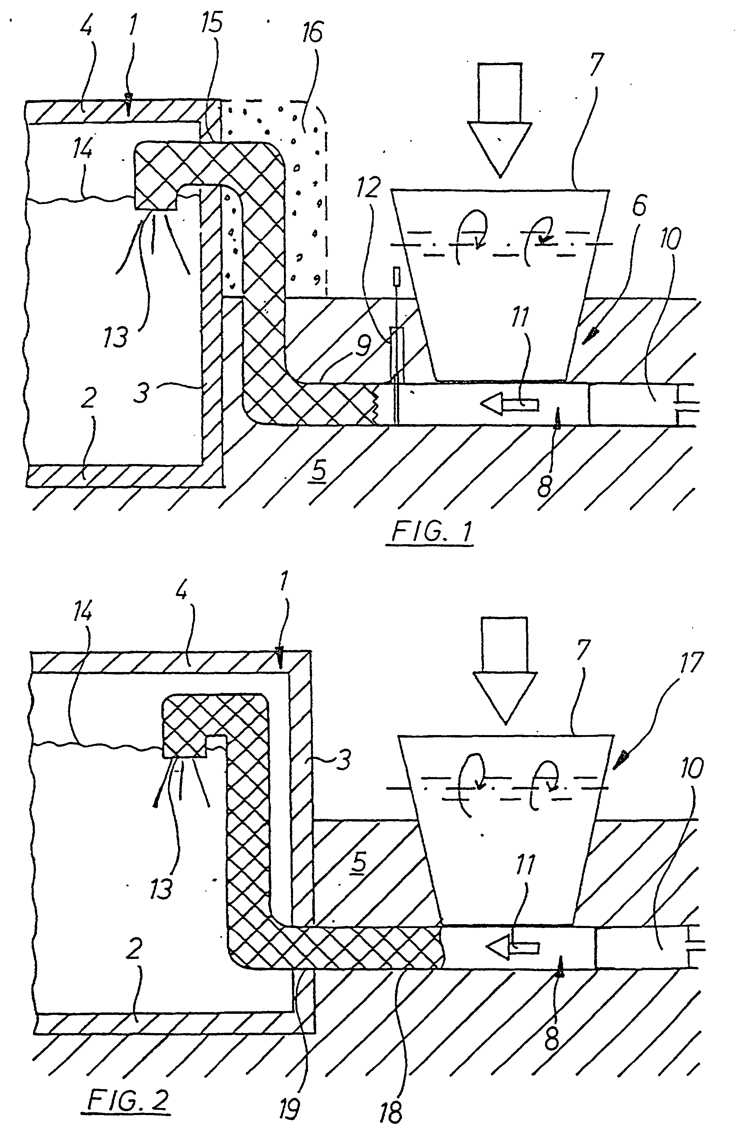

In der Fig. 1 ist schematisch eine teilweise Schnittansicht durch einen Fermenter 1 einer Biogasanlage gezeigt, der einen Behälterboden 2, eine Seitenwand 3 und einen Behälterdeckel 4 aufweist. Der Fermenter 1 ist dabei bis in etwa zur Hälfte der Seitenwand 3 im Boden 5 angeordnet.

Weiter ist eine Feststoff-Zudosier-Vorrichtung 6 vorgesehen, die aus einem als Einwurftrichter ausgebildeten Einwurfschacht 7 für organische Feststoffe, einer am Bodenbereich des Einwurfschachts 7 angebrachten Hydraulikpresse 8 als Linearfördereinrichtung und einem nachgeordneten Förderrohr 9 aufgebaut ist. Die Hydraulikpresse 8 umfasst einen im unteren Bereich des Einwurfschachts 7 liegenden Zwischensammelraum als Zylinder und einen Pressenstempel 10, der vorzugsweise in etwa eine Länge entsprechend der Einwurfschachtlänge aufweist. Über den Pressenstempel 10 können die in den Einwurfschacht 7 eingeworfenen organischen Feststoffe aus dem Zwischensammelraum in und durch das Förderrohr 9 gepresst werden, wie dies in der Fig. 1 schematisch durch den Pfeil 11 dargestellt ist. In der in der Fig. 1 dargestellten Pressenstempelposition können die eingeworfenen organischen Feststoffe in den Zwischensammelraum vor den Pressenstempel 10 gelangen, so dass bei einer anschließenden, in Richtung Förderrohr 9 gerichteten Pressenstempelbewegung eine bestimmte Chargenmenge über das Förderrohr 9 in den Fermenter 1 gefördert wird. Die Hublänge des Pressenstempels 10 ist dabei so gewählt, dass dieser am Ende des Vorwärtshubes die Einwurfschachtlänge abdeckt, so dass kein organisches Feststoffmaterial hinter den Pressenstempel 10 gelangen kann. Bei der anschließenden Rückwärtsbewegung des Pressenstempels 10 wird dann der untere Einwurfschachtbereich wiederum für die nächste Charge von nachrutschenden, organischen Feststoffen freigegeben.

Wie dies aus der Fig. 1 weiter ersichtlich ist, ist im Förderrohr 9 am Übergang zwischen der Hydraulikpresse 8 und dem Förderrohr 9 ein Schieber 12 als Absperreinrichtung angebracht.

Wie dies aus der Fig. 1 weiter ersichtlich ist, endet das Förderrohr 9 mit seiner Auslassöffnung 13 im oberen Fermenterinnenbereich unterhalb eines Flüssigkeitsspiegels 14 im Fermenter 1, wobei das Förderrohr 9 dort mit einer nach unten gerichteten Auslassöffnung 13 umgebogen ist. Das Förderrohr 9 ist dicht durch eine Fermenterzuführöffnung 15 in der Seitenwand 3 des Fermenters 1 im oberen Seitenwandbereich geführt, wobei der außerhalb des Fermenters liegende Förderrohrabschnitt zum Teil frostsicher im Boden 5 und/oder mittels einer Wärmedämmung 16 wärmegedämmt geführt ist.

In der Fig. 2 ist eine alternative Ausführungsform einer Feststoff-Zudosier-Vorrichtung 17 zum Fermenter 1 gezeigt, wobei die Feststoff-Zudosier-Vorrichtung 17 im wesentlichen ähnlich zu der Feststoff-Zudosier-Vorrichtung 6 der Fig. 1 aufgebaut ist. Im Unterschied zu dieser ist hier ein Förderrohr 18 durch eine Fermenterzuführöffnung 19 in einem unteren Seitenwandbereich der Seitenwand 3 des Fermenters 1 geführt, wobei der außerhalb des Fermenters 1 liegende Förderrohrabschnitt frostsicher im Boden 5 geführt ist. Der im Fermenter 1 liegende Förderrohrabschnitt ist frostsicher im Fermenter 1 nach oben geführt.

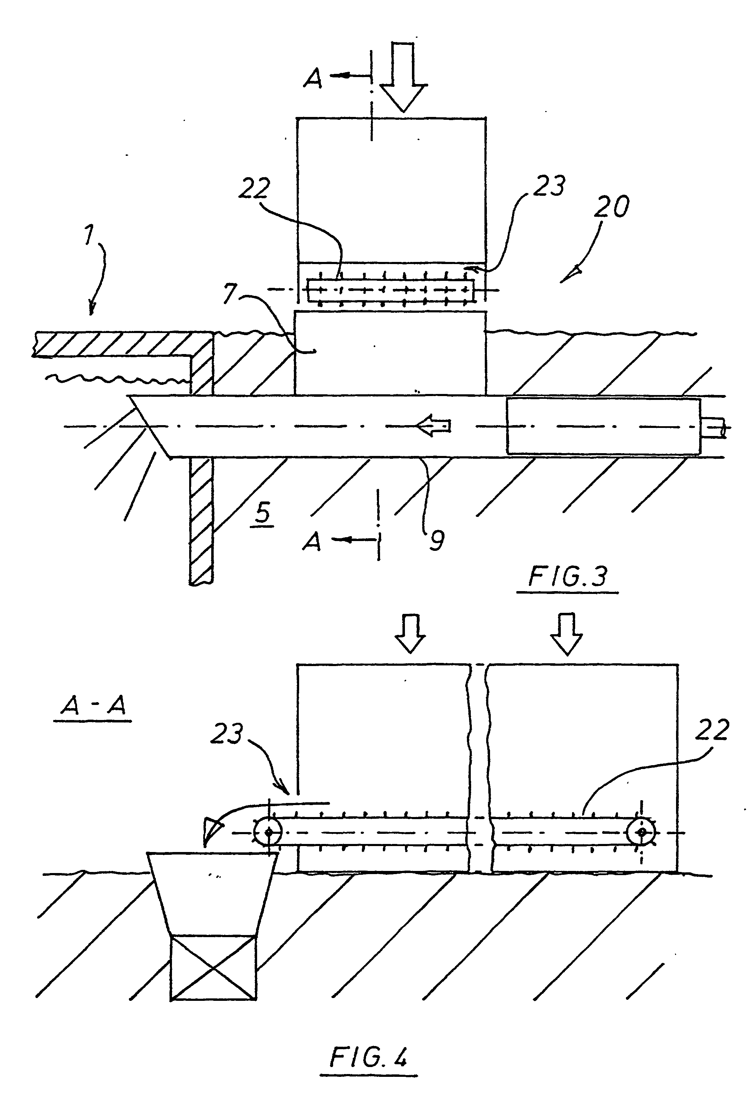

In der Fig. 3 und Fig. 4 ist eine weitere alternative Ausführungsform einer ähnlich aufgebauten Feststoff-Zudosier-Vorrichtung 20 zum Fermenter 1 gezeigt. Der Fermenter 1 ist hier vollständig im Boden 5 angeordnet. Das Förderrohr 9 ist hier gerade in den Fermenter 1 geführt und lediglich schräg abgeschnitten. Oberhalb und neben dem Einwurfschacht ist ein relativ großer Vorratsbehälter 21 angeordnet mit einer Zuführeinrichtung zum Einwurfschacht 7 in der Art eines Kratzbodens 22. Damit können Feststoffe aus einem Behälterspalt 23 gesteuert heraus und in den Einwurfschacht 7 befördert werden.

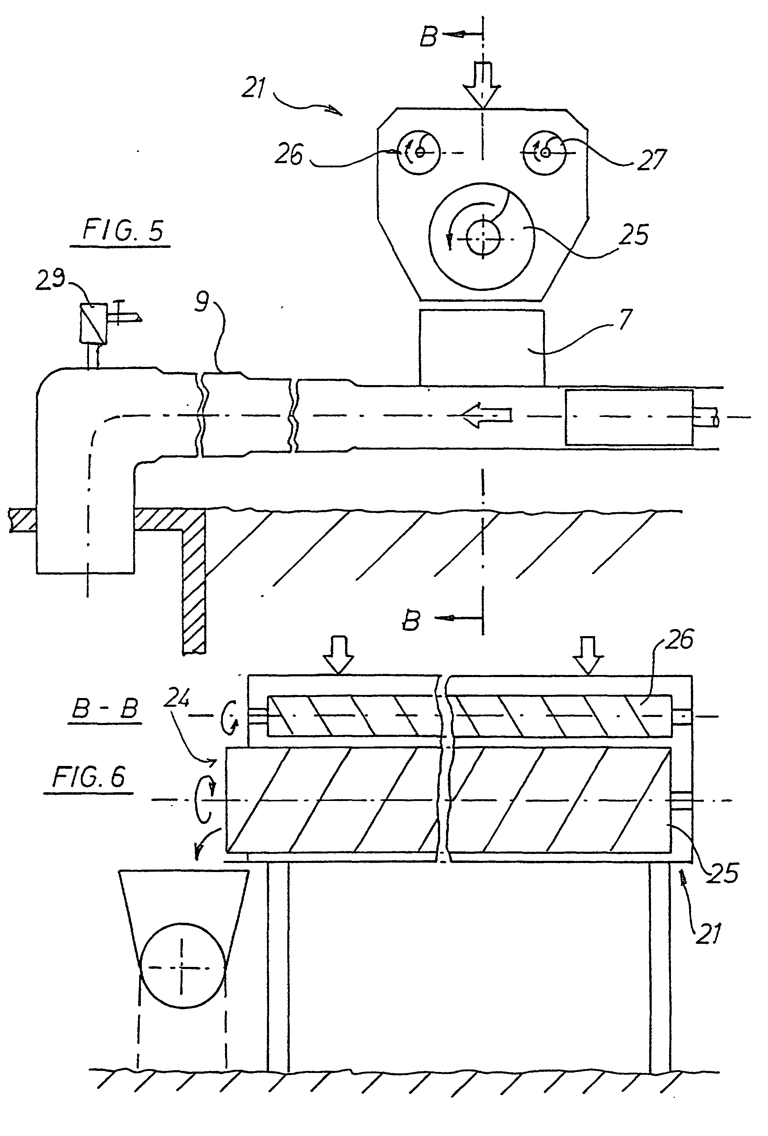

Die Fig. 5 und Fig. 6 zeigen eine alternative Ausführungsform mit einem Vorratsbehälter 21. Dieser enthält eine Zerkleinerungs- und Mischeinrichtung 25, bestehend aus einer Förderschnecke 21 und achsparallel angeordneten Zusatzschnecken 26, 27. Mit der Förderschnecke 21 können Feststoffe aus einer zugeordneten Behälteröffnung 24 gesteuert dem Einwurfschacht 7 zugeführt werden.

Der Durchmesser des Förderrohrs 9 nimmt hier zur Verbesserung der Förderfähigkeit in Richtung auf den Fermenter 1 gestuft zu. Zudem ist für den gleichen Zweck über ein Steuerventil 28 eine Wasser-Spüldüse 29 am Förderrohr 9 ansteuerbar.

## Patentansprüche

1. Feststoff-Zudosier-Vorrichtung an einem Fermenter einer Biogasanlage,
mit einem Fermenterbehälter mit mindestens einer Fermenterzuführöffnung, über die organische Feststoffe in eine im Fermenterbehälter aufgenommene Flüssigkeit zudosiert werden,
**dadurch gekennzeichnet,**
**dass** die Feststoff-Zudosier-Vorrichtung (6; 17; 20) aus einem Einwurfschacht (7) mit anschließendem Zwischensammelraum für organische Feststoffe und einem nachgeordneten Förderrohr (9; 18) sowie einer Linearfördereinrichtung besteht,
**dass** das Förderrohr (9; 18) dicht mit einer Fermenterzuführöffnung (15; 19) verbunden ist, und
**dass** die Linearfördereinrichtung aus einer Kolbenpresse (8) besteht, deren Pressenstempel (10) im Bereich des Zwischensammelraums angetrieben bewegt wird dergestalt, dass für eine chargenweise Förderung im Zwischensammelraum enthaltene organische Feststoffe mittels des Pressenstempels (10) in und durch das Förderrohr (9; 18) gepresst und in die Flüssigkeit im Fermenterbehälter eingebracht werden.

2. Feststoff-Zudosier-Vorrichtung nach Anspruch 1, **dadurch gekennzeichnet, dass** das Förderrohr (9; 18) mit seiner Auslassöffnung (13) im oberen Fermenterinnenbereich oberhalb und/oder unterhalb des Flüssigkeitsspiegels (14) endet und vorzugsweise dort mit einer nach unten gerichteten Auslassöffnung (13) unter den Flüssigkeitsspiegel (14) umgebogen ist.

3. Feststoff-Zudosier-Vorrichtung nach Anspruch 2, **dadurch gekennzeichnet, dass** das Förderrohr (9) durch eine Fermenterzuführöffnung (15) in einer Seitenwand (3) des Fermenterbehälters (1) im oberen Seitenwandbereich oder durch eine Deckenwand geführt ist und der außerhalb des Fermenterbehälters (1) liegende Förderrohrabschnitt frostsicher im Boden (5) und/oder wärmegedämmt geführt ist.

4. Feststoff-Zudosier-Vorrichtung nach Anspruch 2, **dadurch gekennzeichnet,**
**dass** das Förderrohr (18) durch eine Fermenterzuführöffnung (19) in einem unteren Seitenwandbereich und im Fermenterbehälter (1) nach oben geführt ist, und
**dass** der außerhalb des Fermenterbehälters (1) liegende Förderrohrabschnitt frostsicher im Boden (5) und/oder wärmegedämmt geführt ist.

5. Feststoff-Zudosier-Vorrichtung nach einem der Ansprüche 1 bis 4, **dadurch gekennzeichnet, dass** der Durchmesser des Förderrohrs (9) ausgehend von der Kolbenpresse (8) zum Fermenter (1) hin größer wird.

6. Feststoff-Zudosier-Vorrichtung nach einem der Ansprüche 1 bis 5, **dadurch gekennzeichnet, dass** im Verlauf des Förderrohrs (9) wenigstens eine Wasser-Spüldüse (29) für die Anfeuchtung eines im Förderrohr (9) enthaltenen Pfropfens aus organischen Feststoffen angebracht ist.

7. Feststoff-Zudosier-Vorrichtung nach einem der Ansprüche 1 bis 6, **dadurch gekennzeichnet, dass** die Kolbenpresse eine Hydraulikpresse (8) ist.

8. Feststoff-Zudosier-Vorrichtung nach einem der Ansprüche 1 bis 7, **dadurch gekennzeichnet, dass** der Pressenstempel (10) wenigstens eine Länge entsprechend der Länge des Zwischensammelraums aufweist.

9. Feststoff-Zudosier-Vorrichtung nach einem der Ansprüche 1 bis 8, **dadurch gekennzeichnet, dass** im Einwurfschacht (7) oberhalb der Kolbenpresse (8) und gegebenenfalls mit dieser zusammenwirkend wenigstens eine angetriebene Zerkleinerungs- und/oder Mischeinrichtung (25) angeordnet ist.

10. Feststoff-Zudosier-Vorrichtung nach einem der Ansprüche 1 bis 9, **dadurch gekennzeichnet, dass** ein Vorratsbehälter (21) für größere Mengen an organischen Feststoffen vorgesehen ist mit einer Zuführeinrichtung (22; 25) zum Einwurfschacht (7) und zum Zwischensammelraum.

11. Feststoff-Zudosier-Vorrichtung nach Anspruch 10, **dadurch gekennzeichnet, dass** die Zuführeinrichtung aus einer Bodenfördereinrichtung (22) am Boden des Vorratsbehälters (21) besteht, mit der durch einen angeordneten Behälterspalt (23) organische Feststoffe unmittelbar oder mittelbar über eine weitere Fördereinrichtung dem Einwurfschacht (7) zuführbar sind.

12. Feststoff-Zudosier-Vorrichtung nach Anspruch 10, **dadurch gekennzeichnet, dass** der Vorratsbehälter eine Zerkleinerungs- und/oder Mischeinrichtung enthält, die aus einer Förderschnecke (25) und aus wenigstens weiteren zwei, jeweils seitlich der Förderschnecke (25) achsparallel angeordneten Zusatzschnecken (26, 27) mit einer Drehrichtung entgegen der Förderschneckendrehrichtung besteht, und
dass mittels der Förderschnecke (25) durch eine zugeordnete Behälteröffnung (24) organische Feststoffe unmittelbar oder mittelbar über eine weitere Fördereinrichtung dem Einwurfschacht (7) zuführbar sind.

13. Feststoff-Zudosier-Vorrichtung nach einem der Ansprüche 1 bis 12, **dadurch gekennzeichnet, dass** im Förderrohr (9) vorzugsweise am Übergang zwischen der Kolbenpresse (8) und dem Förderrohr (9) wenigstens eine betätigbare Absperreinrichtung (12), insbesondere als Schieber, angebracht ist.

14. Feststoff-Zudosier-Vorrichtung nach einem der Ansprüche 1 bis 13, **dadurch gekennzeichnet, dass** eine programmierbare Steuereinheit vorgesehen ist, mit der in Abhängigkeit von erfassten Betriebsparametern, wie dem Gasdruck und die Temperatür im Fermenterbehälter der Antrieb für den Pressenstempel (10) und gegebenenfalls wenigstens eine Absperreinrichtung (12) und wenigstens eine Spüldüse (29) ansteuerbar sind.

## Claims

1. Apparatus for the metered addition of solid material to a fermenter of a biogas plant,
with a fermenter tank with at least one fermenter feed opening, via which organic solid materials are metered into the liquid held in the fermenter tank,
**characterized**
**in that** the apparatus for the metered addition of solid material (6; 17; 20) comprises a feed shaft (7) with an adjoining intermediate collecting space for organic solid materials and a downstream conveying tube (9; 18) and also a linear conveying device,
**in that** the conveying tube (9; 18) is connected in a sealed manner to a fermenter feed opening (15; 19), and
**in that** the linear conveying device comprises a piston press (8), the press ram (10) of which is moved in a driven manner in the region of the intermediate collecting space in such a way that organic solid materials contained in the intermediate collecting space for conveyance in batches are pressed by means of the press ram (10) into and through the conveying tube (9; 18) and are introduced into the liquid in the fermenter tank.

2. Apparatus for the metered addition of solid material according to Claim 1, **characterized in that** the conveying tube (9; 18) ends with its outlet opening (13) in the upper region inside the fermenter above and/or below the liquid level (14) and is preferably bent round there with a downwardly directed outlet opening (13) under the liquid level (14).

3. Apparatus for the metered addition of solid material according to Claim 2, **characterized in that** the conveying tube (9) is led through a fermenter feed opening (15) in a side wall (3) of the fermenter tank (1) in the upper side wall region, or through a top wall, and the conveying tube portion that lies outside the fermenter tank (1) is led in such a way that it is protected from frost in the ground (5) and/or in a thermally insulated manner.

4. Apparatus for the metered addition of solid material according to Claim 2, **characterized**
**in that** the conveying tube (18) is led through a fermenter feed opening (19) in a lower side wall region and upwards in the fermenter tank (1), and
**in that** the conveying tube portion that lies outside the fermenter tank (1) is led in such a way that it is protected from frost in the ground (5) and/or in a thermally insulated manner.

5. Apparatus for the metered addition of solid material according to one of Claims 1 to 4, **characterized in that** the diameter of the conveying tube (9) becomes greater from the piston press (8) to the fermenter (1).

6. Apparatus for the metered addition of solid material according to one of Claims 1 to 5, **characterized in that** at least one water rinsing nozzle (29) for the moistening of a plug of organic solid materials that is contained in the conveying tube (9) is fitted along the length of the conveying tube (9).

7. Apparatus for the metered addition of solid material according to one of Claims 1 to 6, **characterized in that** the piston press is a hydraulic press (8).

8. Apparatus for the metered addition of solid material according to one of Claims 1 to 7, **characterized in that** the press ram (10) is at least of a length corresponding to the length of the intermediate collecting space.

9. Apparatus for the metered addition of solid material according to one of Claims 1 to 8, **characterized in that** at least one driven comminuting and/or mixing device (25) is arranged in the feed shaft (7), above the piston press (8) and, if appropriate, such that it interacts with the latter.

10. Apparatus for the metered addition of solid material according to one of Claims 1 to 9, **characterized in that** a storage tank (21) for relatively large amounts of organic solid materials is provided, with a feeding device (22; 25) to the feed shaft (7) and to the intermediate collecting space.

11. Apparatus for the metered addition of solid material according to Claim 10, **characterized in that** the feeding device comprises a bottom feeding device (22) at the bottom of the storage tank (21), with which organic solid materials can be fed to the feed shaft (7) through an arranged tank gap (23) directly or indirectly by means of a further conveying device.

12. Apparatus for the metered addition of solid material according to Claim 10, **characterized in that** the storage tank contains a comminuting and/or mixing device, which comprises a conveying screw (25) and at least a further two additional screws (26, 27), respectively arranged axially parallel to the sides of the conveying screw (25) and with a direction of rotation opposite to the direction of rotation of the conveying screw, and
**in that**, by means of the conveying screw (25), organic solid materials can be fed to the feed shaft (7) through an assigned tank opening (24) directly or indirectly by means of a further conveying device.

13. Apparatus for the metered addition of solid material according to one of Claims 1 to 12, **characterized in that** at least one operable shut-off device (12), in particular in the form of a slide, is fitted in the conveying tube (9), preferably at the transition between the piston press (8) and the conveying tube (9).

14. Apparatus for the metered addition of solid material according to one of Claims 1 to 13, **characterized in that** a programmable control unit is provided, with which the drive for the press ram (10) and, if appropriate, at least one shut-off device (12) and at least one rinsing nozzle (29) can be activated in dependence on sensed operating parameters, such as the gas pressure and the temperature in the fermenter tank.

## Revendications

1. Dispositif d'alimentation en matières solides sur un fermenteur d'une installation de biogaz, comprenant un récipient fermenteur avec au moins une ouverture d'arrivée de fermenteur, par laquelle des matières solides organiques sont amenées de façon dosée dans un liquide réceptionné dans le récipient fermenteur, **caractérisé en ce que**, le dispositif d'alimentation en matières solides (6 ; 17 ; 20) comprend un puits d'injection (7) avec un espace collecteur intermédiaire consécutif pour des matières solides organiques et un tuyau transporteur (9 ; 18) placé en aval et un appareil transporteur linéaire, **en ce que** le tuyau transporteur (9 ; 18) est relié de façon étanche à une ouverture d'arrivée du fermenteur (15 ; 19), et **en ce que** le système transporteur linéaire comprend une presse à piston (8), dont le coulisseau (10) est déplacé entraîné dans la zone de l'espace collecteur intermédiaire de telle sorte que des matières solides organiques contenues dans l'espace collecteur intermédiaire sont comprimées au moyen du coulisseau de presse (10) et à travers le tube transporteur (9 ; 18) pour un transport par charge et sont introduites dans le liquide dans le récipient fermenteur.

2. Dispositif d'alimentation de matières solides selon la revendication 1, **caractérisé en ce que** le tuyau transporteur (9 ; 18) se termine avec son ouverture de sortie (13) dans la zone intérieure supérieure du fermenteur au-dessus et/ou au-dessous du niveau de liquide (14) et est recourbé ici de préférence avec une ouverture de sortie (13) dirigée vers le bas au-dessous du niveau de liquide (14).

3. Dispositif d'alimentation en matières solides selon la revendication 2, **caractérisé en ce que** le tuyau transporteur (9) est guidé par une ouverture d'arrivée du fermenteur (15) dans une paroi latérale (3) du récipient fermenteur (1) dans la zone supérieure de la paroi latérale ou par une paroi de plafond et la partie de tuyau transporteur situé à l'extérieur du récipient fermenteur (1) est guidée à l'abri du gel dans le fond (5) et/ou avec une isolation thermique.

4. Dispositif d'alimentation en matières solides selon la revendication 2, **caractérisé en ce que** le tuyau transporteur (18) est guidé vers le haut par une ouverture d'arrivée du fermenteur (19) dans une zone inférieure de paroi latérale et dans le récipient fermenteur (1), et **en ce que** la partie du tuyau transporteur situé à l'extérieur du récipient fermenteur (1) est guidée à l'abri du gel dans le fond (5) et/ou avec une isolation thermique.

5. Dispositif d'alimentation en matières solides selon l'une quelconque des revendications 1 à 4, **caractérisé en ce que** le diamètre du tuyau transporteur (9) devient plus grand à partir de la presse à piston (8) en direction du fermenteur (1).

6. Dispositif d'alimentation en matières solides selon l'une quelconque des revendications 1 à 5, **caractérisé en ce que** dans le tracé du tuyau transporteur (9) est placée au moins une buse de lavage à eau (29) pour l'humidification d'un bouchon contenu dans le tuyau transporteur (9) à base de matières solides organiques.

7. Dispositif d'alimentation en matières solides selon l'une quelconque des revendications là 6, **caractérisé en ce que** la presse à piston est une presse hydraulique (8).

8. Dispositif d'alimentation en matières solides selon l'une quelconque des revendications là 7, **caractérisé en ce que** le coulisseau de presse (10) présente au moins une longueur correspondant à la longueur de l'espace collecteur intermédiaire.

9. Dispositif d'alimentation en matières solides selon l'une quelconque des revendications 1 à 8, **caractérisé en ce qu'**au moins un dispositif entraîné de broyage et/ou de mélange (25) est disposé dans le puits d'injection (7) au-dessus de la presse à piston (8) et éventuellement coopérant avec celle-ci.

10. Dispositif d'alimentation en matières solides selon l'une quelconque des revendications 1 à 9, **caractérisé en ce qu'**un récipient de stockage (21) est prévu pour des quantités assez grandes de matières solides organiques avec un dispositif d'amenée (22 ; 25) pour le puits d'injection (7) et pour l'espace collecteur intermédiaire.

11. Dispositif d'alimentation en matières solides selon la revendication 10, **caractérisé en ce que** le dispositif d'amenée comprend un appareil de transport de fond (22) sur le fond du récipient de stockage (21), avec lequel des matières solides organiques peuvent être amenées directement ou indirectement par un autre système transporteur au puits d'injection (7) à travers une fente de récipient (23) mise en place.

12. Dispositif d'alimentation en matières solides selon la revendication 10, **caractérisé en ce que** le récipient de stockage contient un appareil de broyage et/ou de mélange, qui comprend une vis sans fin de transport (25) et au moins deux autres vis sans fin supplémentaires (26, 27) disposées de façon parallèle, et respectivement sur le côté de la vis sans fin de transport (25) avec un sens de rotation contraire au sens de rotation de la vis sans fin de transport, et **en ce que** des matières solides organiques peuvent être amenées indirectement ou directement au moyen d'un autre système transporteur au puits d'injection (7) au moyen de la vis sans fin de transport (25) à travers une ouverture de récipient (24) attribuée.

13. Dispositif d'alimentation en matières solides selon l'une quelconque des revendications 1 à 12, **caractérisé en ce que** au moins un dispositif d'obturation pouvant être actionné, conçu en particulier comme robinet-vanne, est disposé dans le tuyau transporteur (9) de préférence sur la transition entre la presse à piston (8) et le tuyau transporteur (9).

14. Dispositif d'alimentation en matières solides selon l'une quelconque des revendications 1 à 13, **caractérisé en ce qu'**il est prévu une unité de commande programmable, avec laquelle l'entraînement pour le coulisseau de presse (10) et éventuellement au moins un appareil d'obturation (12) et au moins une buse de lavage (29) peuvent être actionnés en fonction de paramètres de service enregistrés comme la pression de gaz et la température dans le récipient fermenteur.
